# EUROPEAN PATENT APPLICATION

(11) **EP 2 308 397 A1**
(43) Date of publication of application: **13.04.2011**
(21) Application number: 09012714.3
(22) Date of filing: 07.10.2009
(51) Int. Cl.: A61B 17/225, A61N 7/00, G10K 11/30

(54) **Applicator head for emitting shockwaves**

(71) Applicant: Dornier MedTech Systems GmbH, 82234 Wessling (DE)
(72) Inventor: Eizenhoefer, Harald, 82229 Seefeld (DE)
(74) Representative: Grünecker, Kinkeldey, Stockmair & Schwanhäusser Anwaltssozietät

(57) **Abstract**

The invention relates to an application head (1) for emitting shock waves, the applicator head having a focusing means (2) disposed between a shock wave generator (3) and an outlet means (4), the outlet means comprising a therapy surface (5) through which acoustic waves generated by the shock wave generator exit the applicator head. The disclosure has the object to provide an improved applicator head with a good and simple design. This is solved by an applicator head wherein the focusing means is integrally formed with the outlet means.

## Description

The present invention relates to an applicator head for emitting shockwaves having the features of the preamble of claim 1.

The therapy devices used for medical techniques using shockwaves comprise a shock wave generator for generating an acoustic pressure pulse or wave, as well as some sort of acoustical focusing means in order to pass a resulting focused shockwave into a patient's body.

A prior art orthopedic shockwave device is known using an electromagnetic shockwave emitter (EMSE) as shockwave generator for generating a pressure pulse, and a focusing means such as an acoustical lens is disposed in front of the EMSE inside a housing. The lens is surrounded by a liquid such as water which serves both as a coolant and as an acoustic transmission medium. The liquid is continuously flushed in order to dissipate heat and remove gas bubbles therein from the applicator head. An acoustic wave or pulse generated by the shockwave generator propagates into and through the transmission medium towards the focusing means, is focused by the focusing means, and further propagates through another layer of the transmission medium towards an outlet means having a high acoustic transmittance. The focusing means, an acoustic lens, is formed of a material having a different, e.g. lower, internal sound-propagation velocity than the surrounding liquid and the body tissue of the patient. The liquid such as water and the body tissue have similar sound-propagation velocities. The outlet means is a cap at a front end of an applicator head and is made of a thermoplastic material, such as polymethylpentene (PMP), because such thermoplastic materials have good acoustic transmission properties while the internal sound propagation velocity is close to that of water or body tissue, which is particularly about 1500 meters per second.

In order to provide the different internal sound-propagation velocity, the lens is made of a different material than the front cap, such as elastomer, e.g. silicone elastomer, which materials have a lower internal sound-propagation velocity, i.e. about 950 meters per second, while rendering an acoustic impedance similar to that of water or body tissue. This ensures minimized reflections between boundary surfaces, and provides a good transmittance.

It is an object of the present invention to provide an improved applicator head for emitting shockwaves with a good and simple design.

This object is solved by an applicator head having the features of independent claim 1.

An acoustic wave generated by the shockwave generator passes through the coolant/transmission liquid in front of the shockwave generator, then enters the integrally formed focusing and outlet means, and is refracted therein. It exits the applicator head when it leaves the integrally formed focusing means and outlet means.

A significant advantage that has been observed using the integrally formed focusing and outlet means is that the yield or efficiency of the acoustic transmission between the shock wave generator and the therapy surface may improve by an increase of acoustic energy in the shock waves leaving the applicator head. This may be in some cases an increase of about 30% of acoustic energy. It is assumed that this effect is caused by the omission of two boundary surfaces on the propagation path of the shock wave inside the applicator head. In the old design of the prior art, however, a focused shock wave crosses a first boundary surface between the focusing means and the transmission liquid, as well as a second boundary surface between the transmission liquid and the outlet means and loses a certain amount of acoustic energy upon crossing said boundary layers.

In the described prior art devices, the durability of the prior art applicator heads can be reduced due to cavitation occurring within the coolant in front of the front cap. Cavitation occasionally occurs, which may cause cavitation pitting on the material of the front cap, eventually eroding an inner surface thereof. Such eroded thermoplastic front caps may then become prone to a high wear and tear, such as breaking.

The problem of cavitation in front of the outlet means is inhibited and the outlet means becomes less prone to cavitation pitting because the focusing means is integrally formed with the outlet means so that the acoustic waves or shockwaves, once focused, exit the applicator head. Then, the shockwaves enter the patient's body, against which the therapy surface of the applicator head rests during therapy.

In one embodiment, the therapy surface of the integrally formed outlet means and focusing means may delimit the applicator head at a therapy side. The therapy side is that side of the applicator head which faces the region of the patient's body that is to be treated. The shockwaves generated in the applicator head exit the applicator head at the therapy side. This has the advantage that the shock waves can directly enter the patient's body once they exit the applicator head and the integrally formed outlet means and focusing means may be directly applied to the patient's body.

In a further embodiment, at least a central passage region of the focusing means and the outlet means, through which acoustic waves can pass, may consist of a material having a homogeneous texture. Homogeneous texture means that at least said central passage region may be formed without inclusions or cavities, so that an acoustic wave or pulse can pass through said region in such a manner that reflections inside said passage region may be minimized or inhibited. This can improve the transmittance of the integrally formed focusing and outlet means, at least in the area thereof through which the acoustic waves pass.

Advantageously, the focusing means and the integrally formed outlet means may be solid. This may provide a good acoustic transmission of an acoustic wave to the therapy surface.

Advantageously, the focusing means and the outlet means may be formed as a bi-convex lens. This may provide for a stronger refraction power than plano-convex lenses may provide, and can further form a therapy surface at one side in an easy manner. Depending on the lens material and its internal sound-propagation velocity, lenses with concave surfaces may also be rendered by the integrally formed focusing means and outlet means.

In a preferable embodiment, the lens may have different radii of curvature and/or at least one aspherical curvature on the therapy surface and/or at the inner surface. This means that the surface of the focusing means facing towards the shock wave generator may have a stronger curvature than the therapy surface facing towards a patient's body. This may allow for an easy adaption of the refraction power of the integrally formed focusing means and outlet means. Aspherical curvatures may be used to compensate for thick lens errors such as aberration.

In one embodiment, the therapy surface of the outlet means may comprise a curved surface portion, from which a flat surface portion extends. This may have the advantage that the inner features of the applicator head may be wider than the curved surface portion of the therapy portion.

Advantageously, the material of the focusing means and the outlet means may comprise an elastomer. Elastomers such as silicone rubber are resilient and robust, making them less prone to cavitation pitting than typically used thermoplastic materials, such as polymethylpentene (PMP). Due to the resiliency of elastomeric materials, the outlet means may be made resistant to impacts, for instance if an applicator head is dropped. Thermoplastic members may be prone to breaking when a dropped applicator head collides with the ground or with other devices and are therefore less desirable.

In a preferred embodiment, a part of the outlet means may form a lateral impact protector for the applicator head. While additional protective sleeves slipped on the applicator head for providing impact protection may be used to prevent cases in which the applicator head is dropped, an impact protector may be integrally provided so that the number of parts is reduced and the applicator head can be designed slimmer. Further, the number of gaps and crevices can be reduced, which simplifies disinfection of the outlet means.

In a particularly favorable embodiment, the outlet means may extent laterally over an edge of the applicator head to a lateral side of the applicator head. Thereby, the outlet means not only covers the therapy side of the applicator head, but also, at least partly, a lateral side thereof. This allows for a compact design and reduces the number of gaps and crevices of the outlet means. This simplifies the disinfection of the applicator head.

The outlet means may be formed to comprise a sleeve-like extension around at least a lateral part of the circumference of the applicator head. This may have the advantage that a good part of the applicator head may be covered by the outlet means, so that an even more compact and simplified construction is provided.

In one further embodiment, the focusing means may be detachable in conjunction with the outlet means from the applicator head. The number of parts to be disassembled can be reduced for cases when maintenance or repair is required or the user makes a replacement.

In a favorable embodiment, the outlet means may be connected to the applicator head by an engagement, in particular either a frictional engagement and/or form-fit. This method of connecting may allow easy assembly and disassembly of the outlet means with the applicator head. Such connections are easy to manufacture, while also being secure and reliable alone, but especially when combined.

In one preferred embodiment, a fastening member provided with the outlet means may reinforce said engagement. Such a fastening member can reinforce the engagement by pushing one engagement member towards the other and might be, e.g., a seamless ring slipped onto a lateral side of the outlet means in a fashion known from hose connectors. This can ensure a reinforced and secure engagement between the outlet means and the applicator head, in order to inhibit accidental and/or unauthorized detachment of outlet means from the applicator head. Tools might be required to removed or disengage the fastening member.

In an advantageous embodiment, the outlet means may form a sealing with the applicator head. This may allow reducing the number of sealings in order to confine the coolant/transmission liquid inside the applicator head. The outlet means with the integrally formed focusing means can thereby make up at least one side wall of an applicator head's liquid-tight cavity in which the shockwave generator partially surrounded by liquid is disposed.

The features described above, in connection with diverse exemplary embodiments, may be combined to form further preferable embodiments. Two exemplary embodiments of the invention and an alternative of a detail of the invention are shown in the drawings, and are described hereinafter. The following is shown in the drawings:
Fig. 1 shows a schematic cross-sectional side view of a first exemplary embodiment of the inventive applicator head;
Fig. 2 shows a schematic sectional side view of a second exemplary embodiment of the inventive applicator head;
Fig. 3 shows a schematic sectional view of an alternative exemplary outlet means embodiment engaged with an annular protrusion.

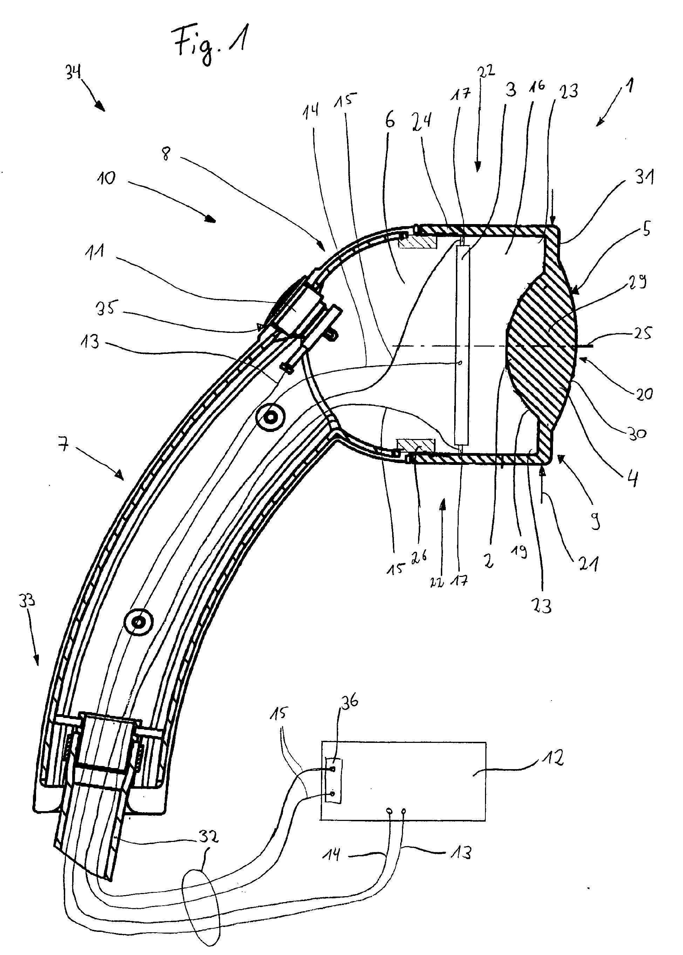

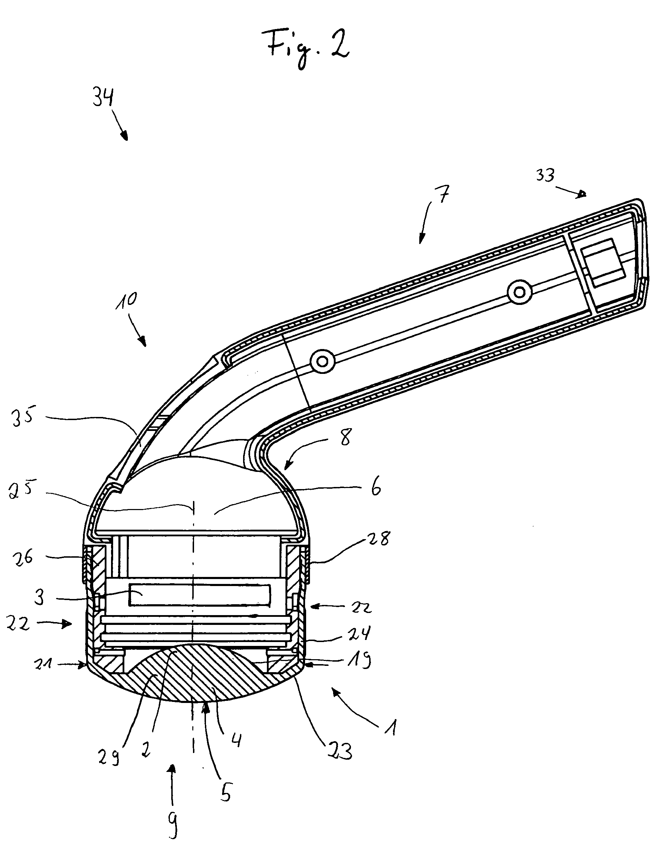

Figs. 1 and 2 exemplary show schematic sectional side views of shockwave therapy devices 34 having applicator heads 1 for emitting shockwaves. The applicator heads 1 each have a focusing means 2 disposed between a shock wave generator 3 and an outlet means 4. The focusing means 2 and the outlet means 4 are integrally formed and can be considered as forming a front cap. The outlet means 4 has a therapy surface 5 through which focused acoustic waves, i.e. shockwaves, generated by the shock wave generator 3 exit the applicator head 1.

The applicator head 1 is exemplary described in the context of hand-held therapy devices 34 having a handle portion 7 extending from the applicator head 1. In both examples of the therapy devices 34, the handle portion 7 extends in an inclined manner from a back side 8 of the applicator head 1 opposing a therapy side 9 of the applicator head 1. The handle portion 7 may be curved as shown in Fig. 1, or generally straight as shown in Fig. 2, or may also assume other shapes. Both examples assume a configuration which might be considered as resembling a shower head; however, designs other than these are also possible. The shock wave generator 3 is disposed inside an internal cavity 6 of the applicator head 1, the cavity being mainly defined by the front cap, i.e. the focusing means 2 integrally formed with the outlet means 4, a sleeve-like extension 24 at a lateral side 22 of the applicator head 1, and the back side 8 of the applicator head 1.

As shown in Fig. 1, at a transition region 10 between the handle portion 7 and the applicator head 1, a trigger 11 is provided which, when pushed, sets off an acoustic wave generated by the shock wave generator 3. The trigger 11 is disposed at a recess 35 in the transition region and functionally connected to a table top unit 12 by means of a signal cable 13. The shock wave generator 3, schematically shown in Figures 1 and 2, is preferably formed as an electromagnetic shock wave emitter (EMSE) which is driven by high electric currents. In order to provide said high electric currents, the table top unit 12 is comprising a high current electrical power source connected to the shock wave generator 3 by means of a power cable 14.

For the purpose of dissipation of heat that occurs inside the applicator head 1 and mainly at the shock wave generator 3, coolant conduits 15 feed and discharge a liquid coolant to and from the applicator head 1, thereby connecting the applicator head 1 with the table top unit 12 in which a heat exchanging device is provided. The coolant liquid at least partially surrounds and circulates around the shock wave generator 3 when being pumped by the heat exchanging device 36.

The signal cable 13, power cable 14 and the coolant conduits 15 are routed through a harness formed by hose 32 which is connected to the handle portion 7 at a far end 33 thereof. Preferably, the signal cable 13 and the power cable 14 comprise individually insulated conductors combined in additional insulating sheaths which electrically and mechanically insulate the electrical conductors from the tubing with the coolant conduits 15.

The shock wave generator 3 schematically shown in Figure 1 and feedthroughs 17 partition the internal cavity 6 to include a liquid-filled space 16 encircled by the outlet means 4, the integrally formed focusing means 2 and the shock wave generator 3 and the feedthroughs 17.

Coolant liquid can enter and exit the liquid-filled space 16 via the feedthroughs 17 to flush said space 16.

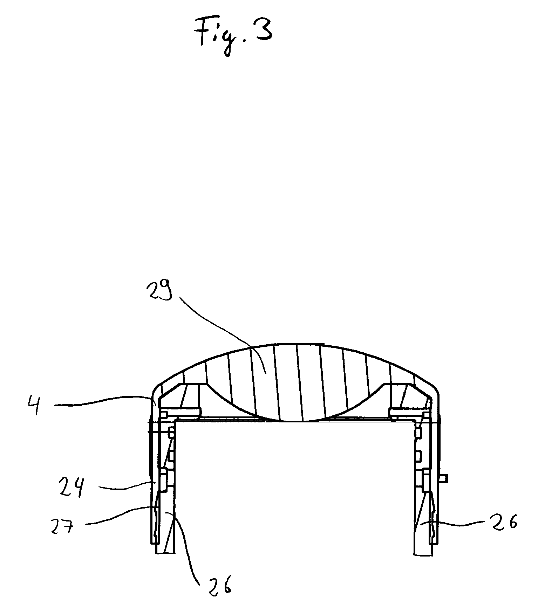

A functionally similar internal configuration may be provided in the embodiment shown in Fig. 2 and the alternative in Fig. 3, but it is omitted for the sake of clarity of the Figure.

As can be seen from all Figures, the therapy surface 5 of the front cap with the integrally formed focusing means 2 and outlet means 4 delimits the applicator head 1 at the therapy side 9, so that the acoustic waves exit the applicator head 1 at the therapy surface 5. The therapy surface 5 is to be directly applied to body tissue of a patient, so that a focused acoustic wave, i.e. a focused shock wave leaving the applicator head 1, can directly enter the patient's body for therapy.

The acoustic waves preferably pass through a central passage region 20 around the axis 25 of the focusing means 2 and the outlet means 4. The central passage region 20 is in alignment with an effective area of the shock wave generator 3, over which area shock waves are generated.

In order to obtain a high transmittance and low unwanted reflections of the acoustic wave inside the applicator head 1, the material making up at least central passage region 20 has a homogenous texture. Homogeneous texture means that the occurrence of inclusions or cavities, which might cause reflections or lower the transmittance, is inhibited in this area. Accordingly, the integrally formed outlet means 4 and focusing means 2 are preferably formed as a solid part of one material having constant properties throughout its dimension.

As shown in all Figures, the front cap including the outlet means 4 extends laterally over a circular edge 23 of the applicator head 1 to a lateral side 22 of the applicator head 1. That part of the front cap, the outlet means 4, respectively, disposed around the lateral side 22 of the applicator head 1 is formed as an integral sleeve-like extension 24 which extends around at least a part of the lateral circumference at the lateral side 22 of the applicator head 1. The sleeve-like extension 24 forms a lateral side wall of the applicator head 1.

The applicator head 1 is formed annular, approximately cylindrical around the axis 25.

The front cap with the integrally formed focusing means 2 and outlet means 4 is formed of an elastomer, preferably a silicone elastomer. Said materials can be considered as having impact-absorbing properties, as these materials are very resilient and robust. Therefore, a part of the outlet means 4 forms a lateral impact protector for the applicator head 1. This applies particularly for the exposed circular edge 23 of the applicator head 1 which edge 23 is exposed and may be protected by the overlapping outlet means 4.

The sleeve like extension 24 forms an impact protection at the lateral side 22 of the applicator head. If one of the applicator heads 1 shown in the figures would collide with an obstacle, at least the applicator head 1 and the means provided therein would be at least partially protected from the impact, while the front cap would not necessarily be subject to breaking. Especially, the outlet means 4 of the applicator head 1 is less prone to breaking than other devices with brittle thermoplastic outlet means and may absorb at least part of the impact.

As the focusing means 2 and the outlet means 4 as well as the extension 24 around the lateral side 22 are integrally formed, that is, as one piece, the focusing means 2 and the outlet means 4 are detachable in conjunction with one another from the applicator head 1. This simplifies procedures for disassembly in case repairs or maintenance of means disposed inside the applicator head 1 is required. Further, a user may be enabled to reconfigure the applicator head 1 on his own when a different type of focusing means 2 is needed for a particular treatment.

The outlet means 4 is connected to the applicator head 1 by an engagement. In particular, the outlet means 4 may be connected to the applicator head 1 by a frictional engagement, as shown in Figs. 1 and 2. Alternatively, a form-fit or a combination of frictional engagement and form-fit is also possible, as shown in Fig. 3. Figs. 1 and 2 schematically show an annular protrusion 26 onto which the outlet means 4 is slipped. The embodiments shown in Figs. 1 and 2 show to secure the outlet means 4 mainly by a frictional engagement between the extension 24 and the annular protrusion 26.

Form-fit engagements in which parts engage one another can also be used. An example of a form-fit engagement is shown in Fig. 3 in which barbs or ridges are disposed on the outside of the annular protrusion 26. If the material of the outlet means 4 is resilient, it is possible that the barbs on one part impress the material on the other part, thereby establishing a form-fit or a combination of form-fit and frictional engagement. It is further possible that the sleeve-like extension 24 be pushed into a recess formed in or at the annular protrusion 26 in order to establish the engagement. Fig. 1, for example, could also be understood as showing a glued connection between the extension 24 and the annular protrusion 26.

The engagement between the outlet means 4 and the applicator head 1 can additionally be reinforced by a fastening member 28 which may advance a portion of the front cap towards a portion of the applicator head 1. For example, it may be a seamless metal ring slipped on the outside of the sleeve-like extension of the outlet means 4, as shown in Fig. 2. An adequate metal material for such a fastening member 28 used in medical applications would be stainless steel or other non-metallic materials might also be usable.

The engagement shown in Figs. 1 to 3 is capable of forming a sealing with the applicator head 1. As shown, the applicator head 1 is flushed with coolant liquid, and the engagement between the outlet means 4 and the applicator head 1 preferably forms a sealing. Thereby, the coolant liquid is confined inside the liquid-filled space 16 of the applicator head 1, as well as the coolant conduits 15 and table top units 12, which are in fluid communication with the applicator head.

The outlet means 4 integrally formed with the focusing means 2 may at least partially form a bi-convex lens portion having a first curvature, i.e. the inner surface 19 of the focusing means 2, and a second curvature, i.e. the therapy surface 5. Depending on the actual requirement in terms of refraction power for the respective application, the curvature of the therapy surface 5 on the outlet means 4 and the curvature on the focusing means 2 may be designed and adapted so that a required refraction power of the bi-convex lens is established. It is possible that the focusing means 2 be formed with a concave inner surface 19 and/or therapy surface 5. The lens 29 may have different radii of curvature on the therapy surface 5 and the inner surface 19 and/or may have at least one aspherical curvature. This can compensate for effects occurring with thick lenses such as aberration. The therapy surface 5 is preferably so constructed that a good abutment against a patient's body tissue is established while the inner surface 19 is adapted to guarantee the desired refraction. In therapy devices 34 used for medical treatment, the focus should be somewhat diffused. The shockwave generator 3 would be positionally fixed relative to the focusing means 2.

The curvature of the therapy surface 5 can also extend over the entire diameter 21 of the applicator head 1 as shown in Figs. 2 and 3.

The shockwaves then enter the patient's body and spread therein, i.e. beyond the focus. This is to trigger the body's own repair mechanisms to initiate natural healing of a variety of medical indications.

Schematically, as shown in Fig. 1, the therapy surface 5 of the outlet means 4 comprises a curved surface portion 30 from which a flat surface portion 31 extends laterally.

During use, the applicator head 1 is either held at the handle portion 7, or the hand of the user grasps the applicator head 1 around the lateral side 22. In the first case, the trigger 11 can be pressed using the user's thumb. In the second case, the index or middle finger might be used to press trigger 11. The therapy surface 5 is applied to a patient's body portion to be treated. Once the trigger 11 is pressed, a signal is routed to the table top unit 12 via the connection of the signal cable 13, the table top unit 12 then transmits power to the shock wave generator 3 via power cable 14. Coolant liquid flows through the coolant conduits 15 and circulates inside the applicator head 1 to cool the shock wave generator 3.

Upon receipt of the power from the table top unit 12, the shock wave generator 3 emits at least one acoustic wave in a direction of the axis 25 and towards the therapy side 9. The acoustic wave enters the coolant between the shock wave generator 3 and the focusing means 2, passes through the coolant and enters the focusing means at the inner surface 19.

Due to the geometry of the outlet means 4 and the focusing means 2 and the different internal sound propagation velocities of the different materials, refraction takes place so that the acoustic wave or pressure pulse may be focused. It then exits the outlet means 4 at the therapy surface 5 as a focused shockwave or focused pressure pulse.

## Claims

1. Applicator head (1) for emitting shock waves,
the applicator head (1) having a focusing means (2) disposed between a shock wave generator (3) and an outlet means (4), the outlet means (4) comprising a therapy surface (5) through which acoustic waves generated by the shock wave generator (3) exit the applicator head (1),
**characterized in that**
the focusing means (2) is integrally formed with the outlet means (4).

2. Applicator head (1) according to claim 1,
**characterized in that**
the therapy surface (5) of the integrally formed focusing means (2) and outlet means (4) delimits the applicator head (1) at a therapy side (9).

3. Applicator head (1) according to one of the preceding claims,
**characterized in that**
at least a central passage region (20) of the focusing means (2) and the outlet means (4), through which acoustic waves can pass, consists of a material having an homogenous texture.

4. Applicator head (1) according to one of the preceding claims,
**characterized in that**
the focusing means (2) and the integrally formed outlet means (4) are solid.

5. Applicator head (1) according to one of the preceding claims,
**characterized in that**
the focusing means (2) and the outlet means (4) form a bi-convex lens.

6. Applicator head (1) according to claim 5,
**characterized in that**
the lens (29) has different radii of curvature and/or at least one aspherical curvature on the therapy surface (5) and/or at the inner surface (19).

7. Applicator head (1) according to one of the preceding claims,
**characterized in that**
the therapy surface (5) of the outlet means (4) comprises a curved surface portion (30) from which a flat surface portion (31) extends.

8. Applicator head (1) according to one of the preceding claims,
**characterized in that**
the material of the focusing means (2) and the outlet means (4) comprises an elastomer.

9. Applicator head (1) according to one of the preceding claims,
**characterized in that**
a part of the outlet means (4) forms a lateral impact protector for the applicator head (1).

10. Applicator head (1) according to one of the preceding claims,
**characterized in that**
the outlet means (4) extends laterally over an edge (23) of the applicator head (1) to a lateral side (22) of the applicator head (1).

11. Applicator head (1) according to claim 10,
**characterized in that**
the outlet means (4) comprises a sleeve-like extension (24) around at least a part of the lateral circumference of the applicator head (1).

12. Applicator head (1) according to one of the preceding claims,
**characterized in that**
the focusing means (2) is detachable in conjunction with the outlet means (4) from the applicator head (1).

13. Applicator head (1) according to one of the claims 10 to 12,
**characterized in that**
the outlet means (4) is connected to the applicator head (1) by an engagement, in particular, a frictional engagement and/or form-fit.

14. Applicator head (1) according to claim 13,
**characterized in that**
a fastening member (28) provided with the outlet means (4) reinforces said engagement.

15. Applicator head (1) according to one of the preceding claims,
**characterized in that**
the outlet means (4) forms a sealing with the applicator head (1).
